(19) Europäisches Patentamt

European Patent Office

Office européen des brevets

(11) Numéro de publication: **0 349 509**
**A1**

(12) **DEMANDE DE BREVET EUROPEEN**

(21) Numéro de dépôt: 89870076.0

(22) Date de dépôt: 29.05.89

(51) Int. Cl.⁵: **A 61 K 47/00**
A 61 K 45/08, A 61 K 9/48,
A 61 K 9/66

(30) Priorité: 07.06.88 LU 87233

(43) Date de publication de la demande:
03.01.90 Bulletin 90/01

(84) Etats contractants désignés:
AT BE CH DE ES FR GB GR IT LI LU NL SE

(71) Demandeur: "PHARLYSE", Société Anonyme
Boulevard Royal, 2
Luxembourg (LU)

(72) Inventeur: Deboeck, Arthur Marie
Bario Navaro Km. 0,9 Box 705
Gurabo Puerto Rico 00658 (US)

Maes, Paul
Rue Porte de Lorette 76
B-4540 Visé (BE)

Baudier, Philippe
Avenue Blucher 10
B-1410 Waterloo (BE)

(74) Mandataire: Schmitz, Yvon et al
Bureau Gevers S.A. rue de Livourne 7 bte 1
B-1050 Bruxelles (BE)

(54) **Composition pharmaceutique à base d'un anti-inflammatoire non stéroidien, son procédé de préparation et son utilisation.**

㊿ Composition pharmaceutique destinée à l'administration par voie orale comprenant un anti-inflammatoire non stéroïdien comme substance pharmacologiquement active et un excipient pharmacologiquement acceptable, l'excipient comprenant au moins une association constituée de glycérides d'acides gras à hydrophilie contrôlée ou de glycérides et de polyglycides d'acides gras à hydrophilie contrôlée, cette association en mélange avec la substance pharmacologiquement active formant un mélange eutectique.

EP 0 349 509 A1

## Description

## Composition pharmaceutique à base d'un anti-inflammatoire non stéroïdien, son procédé de préparation et son utilisation

La présente invention est relative à une composition pharmaceutique à administrer par voie orale, comprenant un anti-inflammatoire non stéroïdien comme substance pharmacologiquement active et un excipient pharmacologiquement acceptable, à son procédé de préparation et à son utilisation.

La difficulté d'obtention d'un mélange homogène de poudres lors de la préparation d'une forme galénique à base d'anti-inflammatoire non stéroïdien (A.I.N.S.) est un problème majeur auquel le formulateur se trouve confronté. En effet, les A.I.N.S. se présentent fréquemment sous la forme de poudres fines de densités apparentes très faibles et présentant des propriétés de cohésion interparticulaires, ce qui rend difficile, voire impossible, leur mise en forme galénique. Un des moyens de contourner ce problème est l'utilisation de la technique de la granulation humide. Ce procédé présente néanmoins l'inconvénient de nécessiter un nombre plus important d'opérations, telles que mélange, mouillage, granulation, séchage et calibrage, ce qui rend le procédé plus long et donc plus coûteux.

Une autre voie d'approche serait la réalisation de préparations liquides soit sous forme de solutions, soit sous forme de suspensions. Ces types de préparations présentent toutefois l'inconvénient de posséder un goût souvent inacceptable ainsi qu'un dosage peu précis au stade de l'utilisation par le patient.

Un moyen de rendre le dosage précis et de masquer complètement le goût serait de placer les solutions ou suspensions d'A.I.N.S. dans des capsules de gélatine molle. Cette technique, étant donné qu'elle permet un masquage complet du goût, permet la réalisation d'un médicament acceptable par le patient. Ce procédé, bien que tout à fait réalisable, présente cependant l'inconvénient d'être d'une grande complexité à mettre en oeuvre. En effet, il requiert une technologie particulière de telle sorte qu'il est réservé à des producteurs spécialisés dans ce domaine. Ces considérations rendent le procédé extrêmement coûteux. Une autre solution, économiquement beaucoup plus avantageuse, consiste à remplacer la capsule de gélatine molle par des gélules ou capsules de gélatine dure utilisées depuis longtemps dans l'industrie pharmaceutique.

Les capsules de gélatine dure permettent le remplissage de poudres, liquides ou pâtes non aqueux. De par le fait qu'elles nécessitent une technologie beaucoup plus simple que pour les capsules molles, elles laissent aux fabricants de produits pharmaceutiques pâteux ou liquides la liberté de réaliser leur production dans leur propre usine avec des coûts de fabrication nettement inférieurs. Outre ces avantages, le milieu pâteux ou liquide dans lequel est incorporé la substance médicamenteuse représente l'approche la plus sûre pour minimiser les écarts de dosage en principe actif qui sont parfois rencontrés quand la matière à remplir est pulvérulente.

Actuellement, ce type de capsules de gélatine dure peuvent être scellées, ce qui les rend étanches et pratiquement inviolables.

Il est également connu que les anti-inflammatoires non stéroïdiens (A.I.N.S.) lors de leur administration à des êtres vivants, présentent l'inconvénient d'entraîner souvent des effets secondaires, tels qu'intolérances ou ulcérations gastro-intestinales.

La présente invention a pour objet de prévoir une composition pharmaceutique destinée à l'administration par voie orale à base d'A.I.N.S. permettant un dosage précis et l'obtention d'une excellente biodisponibilité, tout en diminuant de façon significative les effets secondaires précités rencontrés fréquemment lors de l'administration de ces A.I.N.S.

La composition permettant l'administration orale d'A.I.N.S. comprend, à cet effet, comme excipient pharmacologiquement acceptable, au moins une association constituée de glycérides d'acides gras à hydrophilie contrôlée ou de glycérides et de polyglycides d'acides gras à hydrophilie contrôlée, cette association en mélange avec l'A.I.N.S. formant un mélange eutectique.

Avantageusement, l'association précitée est constituée de glycérides d'acides gras à hydrophilie contrôlée ou d'un mélange de glycérides et de polyglycides d'acides gras à hydrophilie contrôlée, qui peuvent être plus particulièrement les produits de la réaction d'alcoolyse d'huile de palmiste hydrogénée, d'huile de palme hydrogénée, de cire d'abeille partiellement hydroxylée, de polyoxyéthylène glycols de poids moléculaire inférieur à 1500 et d'autres alcools, tels que polyglycols et sorbitol.

Suivant une forme de réalisation particulièrement avantageuse de l'invention, les glycérides d'acides gras et les mélanges de glycérides et de polyglycides partiels d'acides gras sont constitués par des gélucires et l'A.I.N.S. est choisi dans le groupe comprenant l'ibuprofène, l'indométhacine, le naproxène, le kétoprofène, le sulindac et le diclofénac.

Suivant un mode de réalisation particulièrement avantageux, l'association précitée a un point de fusion compris entre 33 et 64°C et une amphiphilie (équilibre hydrophile/lipophile) comprise entre 1 et 18.

Suivant une autre forme de réalisation particulièrement avantageuse de l'invention, la composition pharmaceutique se présente sous la forme d'une dose unitaire ou posologique, telle que gélule ou capsule de gélatine dure.

L'invention se rapporte également à la préparation de cette composition ainsi qu'à son utilisation.

Comme on vient de le mentionner précédemment, la composition pharmaceutique de la présente invention est constituée d'un anti-inflammatoire non stéroïdien comme substance pharmacologiquement active et d'au moins une association de glycérides ou de glycérides et de polyglycides d'acides gras à hydrophilie contrôlée, comme excipient pharmacologiquement acceptable, cette association en mélange avec l'A.I.N.S. permettant l'obtention d'un mélange eutectique, qui peut notamment être conditionné dans des gélules ou capsules de gélatine dure. Cette composition possède, en plus d'un mode de préparation fiable, la caractéristique de permettre une diminution significative des effets secondaires se rencontrant très fréquemment lors de l'administration de préparations pharmaceutiques contenant des principes actifs appartenant à la famille des A.I.N.S., tels qu'intolérances ou ulcérations gastriques. Cette association est de préférence constituée de glycérides ou de glycérides et de polyglycides d'acides gras à hydrophilie contrôlée, qui sont les produits de la réaction d'alcoolyse d'huile de palmiste hydrogénée, d'huile de palme hydrogénée, de cire d'abeille partiellement hydroxylée, de polyoxyéthylène glycols de poids moléculaire inférieur à 1500 et d'autres alcools, tels que polyglycols et sorbitol. Ces glycérides ou mélanges de glycérides et de polyglycides d'acides gras, dont des exemples avantageux sont les gélucires, ont d'une manière générale un point de fusion compris entre 33°C et 64°C et une amphiphilie (équilibre hydrophile/lipophile) comprise entre 1 et 18. Les gélucires sont en fait les produits de la réaction d'alcoolyse du mélange de ces différents composants en diverses proportions, en fonction du point de fusion et de l'hydrophilie recherchés. Comme A.I.N.S. utilisables, on peut citer, suivant l'invention, l'ibuprofène, l'indométhacine, le naproxène, le piroxicam, le kétoprofène, le sulindac, le diclofénac et les mélanges de deux ou plusieurs de ces substances.

Les expressions "dose unitaire" et "dose posologique" telles qu'elles sont utilisées dans le présent mémoire, désignent une dose physiquement déterminée contenant une quantité déterminée de substance pharmacologiquement active, cette quantité étant telle qu'une ou plusieurs doses sont nécessaires pour obtenir un effet avantageux.

D'une façon générale, le mélange eutectique peut être obtenu en incorporant dans les glycérides ou dans le mélange de glycérides et de polyglycides de 1 à 70 % de substance pharmacologiquement active. La quantité de substance active à introduire est bien entendu fonction de la dose à administrer en une fois chez l'individu à traiter, les quantités de glycérides et/ou de polyglycides étant fonction du point de fusion le plus bas recherché. La composition suivant l'invention se présente sous une forme posologique unitaire, avantageusement sous la forme d'une gélule ou capsule de gélatine dure, pour les raisons précitées, dont la dose en substance active a été préétablie de sorte que l'administration de la dose journalière de cette substance pourra se faire de façon très simple.

Ainsi qu'on l'a déjà précisé, la présente invention concerne également un procédé d'obtention de la composition pharmaceutique précitée, qui consiste à mélanger l'A.I.N.S. avec l'association de glycérides ou de glycérides et de polyglycides d'acides gras à hydrophilie contrôlée, à porter ledit mélange à une température dépassant de 10 à 20°C son point de fusion pour le rendre homogène et à porter le mélange eutectique ainsi obtenu à une température légèrement au-dessus de son point de durcissement pour permettre le remplissage précis et exact de gélules ou capsules de gélatine dure, soit par gravité, soit sous pression, la proportion pondérale de la substance pharmacologiquement active à l'association de glycérides ou de glycérides et de polyglycides d'acides gras étant de l'ordre de 1 à 70 %. Chaque gélule ou capsule contiendra environ 20 à 800 mg de mélange eutectique et 5 à 400 mg de substance pharmacologiquement active. Les gélules ou capsules refermées peuvent alors être scellées soit par points ou par banderolage, soit par des solutions en fonction de l'équipement disponible chez le fabricant.

On donne ci-après des exemples non limitatifs de formulations galéniques pouvant être utilisées suivant l'invention. Les caractéristiques thermodynamiques des mélanges obtenus ainsi que les résultats de leurs études pharmacologiques permettent également de comprendre l'intérêt et les avantages de la présente invention.

## Exemple 1

### 1.a Fabrication des gélules
On utilise les ingrédients suivants :

| | |
|---|---|
| Gélucire 44/14 | : 230 g |
| Ibuprofène | : 200 g |

Dans un recipient en acier inoxydable d'une capacité d'environ 1 litre, placé dans un bain d'eau dont la température est maintenue entre 55 et 65°C, on fond le gélucire 44/14. Dans l'huile obtenue, on incorpore l'ibuprofène au moyen d'un mélangeur rapide.

On transvase le mélange chaud dans une machine a remplir les gélules dont la cuve, comportant un agitateur lent, est maintenue à 40 ±2°C. On remplit des gélules n° 1 de 430 mg de mélange, c'est-à-dire de 200 mg d'ibuprofène par gélule. Après refroidissement, les gélules sont fermées et scellées au moyen d'une solution 80-20 alcool-eau.

### 1.b Détermination des paramètres thermodynamiques
Le point de fusion du mélange est déterminé par analyse thermique différentielle.
Point de fusion de l'ibuprofène : 76,5°C
Point de fusion du gélucire 44/14 : 44°C

Point de fusion du mélange : 35°C

On constate donc une forte diminution du point de fusion du mélange par rapport aux points de fusion des composants du mélange, ce qui est la caractéristique d'un mélange eutectique.

1.c Etude pharmacologique

Une étude comparative a été réalisée avec le produit de l'Exemple 1 et un produit se trouvant sur le marché contenant la même quantité d'ibuprofène [Brufen (marque déposée)], chez 15 lapines néozélandaises. Ce groupe de lapines a été divisé par randomisation en 3 groupes de 5 lapines chacuns : un groupe "traitement Liprofen" (mélange de l'Exemple 1), un groupe "traitement Brufen" et un groupe placebo. Les trois préparations ont été administrées aux animaux au moyen d'une sonde gastrique à la dose de 130 mg d'ibuprofène par kg et par jour et pendant 14 jours consécutifs.

L'évolution pondérale des 3 groupes a été analysée car elle est un bon indicateur des troubles gastro-intestinaux.

Evolution pondérale (kg) des 3 groupes de lapines traitées par le Brufen (forme de référence), le produit de l'Exemple 1 et un groupe placebo, pendant 14 jours. Dose d'ibuprofène quotidienne administrée : 130 mg/kg

|  | Jour : 0 | Jour : 14 | Différence de poids |
|---|---|---|---|
| Témoin (placebo) | $1,703 \pm 0,086$ | $2,112 \pm 0,119$ | + 0,409 |
| Exemple 1 | $1,638 \pm 0,093$ | $2,058 \pm 0,180$ | + 0,420 |
| Brufen | $1,707 \pm 0,182$ | $1,922 \pm 0,196$ | + 0,215 |

L'analyse statistique montre qu'avant l'épreuve les poids moyens des 3 lots de lapines ne sont pas significativement différents dans leur ensemble au seuil de 5 %.

Après traitement, l'analyse statistique permet de conclure que les évolutions pondérales du lot témoin et de celui traité par le produit de l'Exemple 1 sont comparables. Par contre, le groupe traité par la forme de référence se différencie statistiquement de celui traité par la forme galénique de l'Exemple 1.

Seules les caractéristiques galéniques différentes des deux préparations peuvent expliquer une réponse différente de la part des animaux étant donné que la quantité d'ibuprofène administrée dans les deux cas est identique.

On sait d'autre part qu'au cours de ses essais de tératogénèse, Adams (Tox. Appl. Pharmacol. 1969, 15, pages 130-330) a remarqué que l'administration journalière de 60 mg/kg d'ibuprofène a des lapines, gravides, ralentissait leur croissance et entraînait la formation d'ulcères gastriques. Cette étude a été répétée par le Prof. J. C. Cazin (Etude d'innocuité par voie orale chez le lapin de soluphen gélule dosée à 200 mg d'ibuprofène) en utilisant une forme galénique d'ibuprofène du marché en comparaison avec le produit de l'Exemple 1 de la présente invention. Les effets relevés lors de cette étude étaient de même nature que ceux relevés par Adams sur la forme galénique d'ibuprofène du marché alors qu'ils n'étaient pas présents pour la forme galénique de l'Exemple 1.

Une étude plus approfondie de ces deux formes a permis de constater que ces deux formes étaient parfaitement bioéquivalentes de telle sorte que la quantité d'ibuprofène résorbée dans les deux cas était identique.

De plus, il est actuellement admis que l'activité ulcérogène de l'ibuprofène est en grande partie d'origine systémique (S.S. Adams - Rheum. Phys. Med. 1970 Suppl. Symposium on Ibuprofen p. 9). Par conséquent, dans la mesure où dans les conditions utilisées, les quantités de principe actif administrées étaient strictement identiques pour les deux traitements testés, il apparaît peu probable que l'activité ulcérogène de l'ibuprofène se soit révélée davantage avec la forme de référence. En réalité, ce sont plutôt les caractéristiques inattendues et imprévisibles de la composition de la présente invention qui jouent un rôle prépondérant dans l'obtention de réponses différentes de la part des animaux.

**Exemple 2**

En procédant comme dans l'Exemple 1, on a utilisé les ingrédients suivants :

| | |
|---|---|
| Gélucire 53/10 | : 230 g |
| Ibuprofène | : 200 g |
| Point de fusion du gélucire 53/10 | : 53°C |
| Point de fusion de l'ibuprofène | : 76,5°C |
| Point de fusion du mélange | : 42°C |

**Exemple 3**

On a utilisé les ingrédients suivants :

| | |
|---|---|
| Indométhacine | : 50 g |
| Gélucire 50/13 | : 450 g |

On procède comme dans l'Exemple 1.

| | |
|---|---|
| Point de fusion de l'indométhacine | : 160°C |
| Point de fusion du gélucire 50/13 | : 50°C |
| Point de fusion du mélange | : 38,2°C |

Les gélules n° 2 sont remplies de 250 mg de mélange, c'est-à-dire de 25 mg d'indométhacine par gélule.

**Exemple 4**

On a utilisé les ingrédients suivants :

| | |
|---|---|
| Gélucire 44/4 | : 60 parties (poids) |
| Kétoprofène | : 40 parties (poids) |

On procède comme dans l'Exemple 1.

| | |
|---|---|
| Point de fusion du gélucire 44/4 | : 44°C |
| Point de fusion du kétoprofène | : 95°C |
| Point de fusion du mélange | : 12,3°C |

Les gélules n° 4 sont remplies de 120 mg de mélange, c'est à dire de 50 mg de kétoprofène par gélule.

**Exemple 5**

On a utilisé les ingrédients suivants

| | |
|---|---|
| Gélucire 44/14 | : 6 parties (poids) |
| Naproxène | : 4 parties (poids) |

On procède comme dans l'Exemple 1.

| | |
|---|---|
| Point de fusion du gélucire 44/14 | : 44,2°C |
| Point de fusion du naproxène | : 155°C |
| Point de fusion du mélange | : 35,5°C |

Les gélules n° 0 long sont remplies de 500 mg de mélange, c'est-à-dire de 200 mg de naproxène par gélule.

| Pertes sanguines fécales provoquées chez le rat par l'administration orale unique de naproxène à la dose de 18 mg/kg : | |
|---|---|
| | Volume de sang éliminé ($\mu$l/48 h). |
| Témoins | 76 ± 8 |
| Anaprax | 285 ± 54 |
| Naprosyne | 254 ± 70 |
| Naproxène | 227 ± 119 |
| Exemple n° 5 | 221 ± 102 |

Anaprax : Sel de sodium sous forme de comprimés (Syntex).

Naprosyne : Comprimés de Naproxène (Syntex).

Il est bien entendu que la présente invention n'est en aucune façon limitée aux formes de réalisation décrites et que bien des modifications peuvent être envisagées sans sortir du cadre du présent brevet.

## Revendications

1. Composition pharmaceutique destinée à l'administration par voie orale comprenant un anti-inflammatoire non stéroïdien comme substance pharmacologiquement active et un excipient pharmacologiquement acceptable, caractérisée en ce que l'excipient comprend au moins une association constituée de glycérides d'acides gras à hydrophilie contrôlée ou de glycérides et de polyglycides d'acides gras à hydrophilie contrôlée, cette association en mélange avec la substance pharmacologiquement active formant un mélange eutectique.

2. Composition suivant la revendication 1, caractérisée en ce que les glycérides d'acides gras ou les glycérides et polyglycides partiels d'acides gras sont les produits de la réaction d'alcoolyse d'huile de palmiste hydrogénée, d'huile de palme hydrogénée, de cire d'abeille partiellement hydroxylée, de polyoxyéthylène glycols de poids moléculaire inférieur à 1500 et d'autres alcools, tels que polyglycols et sorbitol.

3. Composition suivant l'une ou l'autre des revendication 1 et 2, caractérisée en ce que les glycérides ou les glycérides et polyglycides partiels d'acides gras sont un gélucire.

4. Composition suivant l'une quelconque des revendications 1 à 3, caractérisée en ce que l'association précitée a un point de fusion compris entre 33 et 64°C et une amphiphilie comprise entre 1 et 18.

5. Composition suivant l'une quelconque des revendications 1 à 4, caractérisée en ce que la substance pharmacologiquement active est choisie dans le groupe comprenant l'ibuprofène, l'indométhacine, le naproxène, le kétoprofène, le sulindac et le diclofénac.

6. Composition suivant l'une quelconque des revendications 1 à 5, caractérisée en ce que la proportion pondérale de la substance pharmacologiquement active à l'association précitée est de l'ordre de 1 à 70 %.

7. Composition suivant l'une quelconque des revendications 1 à 6, caractérisée en ce qu'elle se présente sous la forme d'une dose unitaire ou posologique.

8. Composition suivant la revendication 7, caractérisée en ce qu'elle se présente sous forme de gélule ou de capsule de gélatine dure.

9. Composition suivant la revendication 8, caractérisée en ce qu'une gélule ou capsule comprend environ 20 à environ 800 mg de mélange eutectique.

10. Composition suivant la revendication 9, caractérisée en ce qu'une gélule ou capsule comprend environ 5 à 400 mg de substance pharmacologiquement active.

11. Procédé de préparation de la composition suivant l'une quelconque des revendications 1 à 10, caractérisé en ce qu'il comprend le mélange de la substance pharmacologiquement active avec l'association de glycérides ou de glycérides et de polyglycides d'acides gras, le chauffage dudit mélange à une température dépassant de 10 à 20°C son point de fusion pour le rendre homogène et le chauffage du mélange eutectique ainsi obtenu à une température légèrement au-dessus de son point de durcissement pour permettre le remplissage précis et exact de gélules ou de capsules de gélatine dure, soit par gravité, soit sous pression, la proportion pondérale de la substance pharmacologiquement active à l'association de glycérides ou de glycérides et de polyglycides d'acides gras étant de l'ordre de 1 à 70 % et la dose unitaire en substance pharmacologiquement active de ladite composition étant de l'ordre de 5 à 400 mg.

12. Utilisation de la composition suivant l'une quelconque des revendications 1 à 11, pour la préparation d'un médicament pour le traitement des états inflammatoires et de la douleur.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| P,X | EP-A-0 279 519  (THE BOOTS CO.) <br> * Page 2, lignes 23-54; page 3, lignes 11-14; page 3, lignes 42-43,53-54; revendications * <br> --- | 1-12 | A 61 K  47/00 <br> A 61 K  45/08 <br> A 61 K  9/48 <br> A 61 K  9/66 |
| X | CHEMICAL ABSTRACTS, vol. 105, no. 8, 25 août 1986, page 368, résumé no. 66339k, Columbus, Ohio, US; P. BURI et al.: "Modulation of the kinetics and of the rate of release by an adequate choice of an excepient: Gelucire example", & BULL. TECH./GATTEFOSSE REP. 1985, 78, 67-73 <br> * Résumé * <br> --- | 1-12 | |
| X | CHEMICAL ABSTRACTS, vol. 107, no. 10, 7 septembre 1987, page 382, résumé no. 83811s, Columbus, Ohio, US; J.R. HOWARD et al.: "Drug release from thermosetting fatty vehicles filled into hard gelatin capsules", & DRUG DEV. IND. PHARM. 1987, 13(6), 1031-45 <br> * Résumé * <br> --- | 1-4,7,8 ,11,12 | |
| X | CHEMICAL ABSTRACTS, vol. 78, no. 4, 29 janvier 1973, page 268, résumé no. 20092v, Columbus, Ohio, US; M. KNOESTER et al.: "Solid-liquid equilibrium of binary mixtures of triglycerides with palmitic and stearic chains", CHEM. PHYS. LIPIDS 1972, 9(4), 309-19 <br> * Résumé * <br> --- | 1-12 | **DOMAINES TECHNIQUES RECHERCHES (Int. Cl.4)** <br><br> A 61 K |
| P,X | EP-A-0 274 870  (T.I.L. MEDICAL LTD) <br> * Exemples 122-127; revendications * <br> ----- | 1-12 | |

Le présent rapport a été établi pour toutes les revendications

| Lieu de la recherche | Date d'achèvement de la recherche | Examinateur |
|---|---|---|
| LA HAYE | 12-10-1989 | BERTE M.J. |